# EUROPEAN PATENT SPECIFICATION

(11) **EP 3 031 415 B1**
(45) Date of publication and mention of the grant of the patent: **31.10.2018**
(21) Application number: 14197226.5
(22) Date of filing: 10.12.2014
(51) Int. Cl.: A61B 17/70

(54) **Coupling assembly and polyaxial bone anchoring device comprising the same**
Kupplungseinheit und polyaxiale Knochenverankerungsvorrichtung damit
Ensemble d'accouplement et dispositif d'ancrage osseux polyaxial comprenant celui-ci

(43) Date of publication of application: 15.06.2016
(73) Proprietor: Biedermann Technologies GmbH & Co. KG, 78166 Donaueschingen (DE)
(72) Inventor: Biedermann, Lutz, 78048 VS-Villingen (DE); Fischer, Bernd, 78199 Bräunlingen (DE); Matthis, Wilfried, 79367 Weisweil (DE)
(74) Representative: Prüfer & Partner mbB Patentanwälte · Rechtsanwälte

(56) References cited:
- EP-A1- 2 687 172
- US-A1- 2013 023 941
- US-A1- 2013 103 098
- US-A1- 2013 150 852
- US-A1- 2014 321 945
- US-B1- 6 248 105
- US-B1- 6 280 442

## Description

The invention relates to a coupling assembly for coupling a rod to a bone anchoring element and to a polyaxial bone anchoring device comprising such a coupling assembly. The coupling assembly comprises a receiving part having a channel for receiving the rod, an accommodation space for accommodating a head of a bone anchoring element and a retainer element for retaining the head of the bone anchoring element in the receiving part. The coupling assembly may also comprise a pressure element configured to exert a pressure force onto the head of the bone anchoring element. The coupling assembly is configured to receive the head of the bone anchoring element from a bottom opening of the receiving part and the retainer element may, for example, snap onto the head of the bone anchoring element such as to retain the same in the accommodation space.

Document US 6,248,105 B1 discloses a device for connecting a longitudinal rod with a pedicle screw. The device comprises a sleeve, a hollow cylindrical insert and a spring chuck. The spring chuck has annular flanges held in a ring-shaped channel near the bottom opening of the sleeve. The spring chuck has a cavity designed to receive the head of the pedicle screw. When the rod is locked by means of a tension screw, pressure is exerted by the rod onto the insert, which engages by means of respectively complementary conical engaging surfaces the spring chuck. The spring chuck is thereby crush-locked onto the head of the pedicle screw. Due to the flanges received in the ring-shaped channel of the sleeve, the spring chuck is held and the head is retained in the sleeve.

Document US 6,280,442 B1 discloses a multi-axial bone screw assembly. The assembly comprises a receiver member, a crown member movably disposed in a lower opening portion of the receiver member, and a retaining member, which defines an aperture smaller than a width of a head of a bone anchor received therein. The retaining member is housed in a groove of the receiver member. The groove extends around the lower opening portion. The retaining member prevents removal of the head from the lower opening portion. The retaining member has a C-shape with a gap allowing the retaining member to snap into the groove from a compressed state. Once inserted into the groove, the retaining member cannot be further expanded.

Document US 8,556,938 B1 discloses a polyaxial bone screw assembly, which has a receiver with a lower cavity cooperating with a lower opening. An upper portion of a shank expands a retaining member in the receiver cavity to capture the shank upper portion in the receiver. Either the retaining member or an insert provide for a friction fit of the shank upper portion in the receiver. Final locking of the polyaxial mechanism is provided by frictional engagement between the shank upper portion and the retaining member. A pre-assembled receiver, retaining member and optional insert may be popped-on or snapped-on to the shank upper portion prior to or after implantation of the shank into a vertebra.

Document US 2013/0103098 A1 discloses a polyaxial bone anchor comprising a shank, a receiver for receiving the upper portion of the shank, an insert disposed within the receiver, an expandable retainer captured within the receiver, and a closure structure for the fixation of a longitudinal connecting member (a rod). The retainer can be moved from a first position in an axial direction to a second position adjacent the lower opening of the receiver. When the retainer is in the second axial position, its radial expansion is hindered.

Document US 2013/0023941 A1 discloses a medical implant comprising a bone anchor shank, a bone anchor receiver for receiving the upper portion of the bone anchor shank, an insert disposed within the receiver, an expandable bone anchor retainer captured within the receiver, and a closure structure for the fixation of a rod. The retainer can be moved from a first position in an axial direction to a second position adjacent the lower opening of the receiver. When the retainer is in the second axial position, its radial expansion is hindered.

Document US 2014/0321945 A1 discloses a bone anchor assembly comprising an anchor, a head member with a lateral opening suitable for use with a rod, and a retaining member. When the head of the anchor is inserted into the head member, the retaining member moves from a first position adjacent the proximal opening of the head member to a second position and then back to the first position. Being in the first position, the retaining member cannot be expanded in a radial direction.

It is an object of the present invention to provide a coupling assembly including a retaining function for a head to be introduced from a bottom opening, wherein the reliability and durability as well as the handling of the assembly is improved.

The object is solved by a coupling assembly for coupling a rod to a bone anchoring element according to claim 1, and by a polyaxial bone anchoring device comprising the coupling assembly according to claim 15. Advantageous aspects and embodiments become apparent from the dependent claims.

According to an aspect of the invention, a coupling assembly includes a receiving part, a retainer element and a locking element. The receiving part has an accommodation space with an opening at one end of the receiving part. A head of an anchoring element can be inserted through the opening to be received within the accommodation space. The retainer element is held in position adjacent the opening by an engagement structure provided at or in the accommodation space.

The retainer element is radially expandable and/or compressible even when held in position by an engagement structure and may thus snap over the head when the head is inserted through the opening. In order to retain the head in the accommodation space of the receiving part, the expansion of the retainer element is prevented or at least hindered by providing a locking element. Thereby, according to one embodiment, the locking element radially partially encompasses an engagement portion of the retainer element in order to hinder expansion thereof. In a non-locked state, a small amount of play between respective engagement potions of the retainer element and the locking element is possible as long as a release of the head is impeded. As a consequence, the retainer element is prevented from releasing the head of the anchoring element.

The locking element is allowed to move between a first position in the accommodation space, wherein it permits the retainer element to expand, and a second position, in which the above described radial locking of the retainer element takes place. In the second position, when the head of the anchoring element is to be final locked, a pressure force oriented towards the bottom opening and exerted directly onto the head by an element other than the retainer element (for example by the rod and/or the locking element itself) adversely presses the head against the retainer element. In other words, the head is locked between the rod and the retainer element, or between the locking element (as a pressure element) and the retainer element, or between all three of these elements.

In this state of final locking, the locking element still impedes radial expansion of the retainer element, while the retainer element is firmly held in position in the accommodation space of the receiving part by virtue of the engagement structure. In one non-limiting exemplary embodiment, the engagement structure holding the retainer element is an annular groove formed in the accommodation space while allowing expansion of the retainer element, when the locking element is in the first position, wherein the head of the anchoring element may be inserted into the opening of the accommodation space.

Since the retainer element is supported by the engagement structure in the accommodation space of the receiving part as well as by the radial engagement of the locking element whereas it does not take part in exertion of the pressure force from the rod or another element such as a pressure element, its function is restricted to retaining the head of the anchoring element. Accordingly, its reliability and durability is enhanced.

Further, as the retainer element does not need to fully encompass the head of the anchoring element unlike in above described document US 6,248,105 B1, its size particularly in the direction along the central axis of the receiving part (as seen when being held in position) is considerably reduced such that the retainer element may be loaded into the accommodation space from a top direction opposite the opening of the accommodation space. Hence, in situ assembly of the parts is improved and further narrowing the width of the bottom opening to almost the diameter of the head is made possible. As a consequence, the stability, reliability and durability of the retainer element is further increased, and dimensions of the receiving may be reduced.

The invention will be better understood from the description of various embodiments taken in conjunction with the accompanying drawings. In the drawings:
- Fig. 1: shows a perspective exploded view of a first embodiment of a polyaxial bone anchoring device.
- Fig. 2: shows a perspective view of the bone anchoring device of Fig. 1 in an assembled state.
- Fig. 3: shows a cross-sectional view of the bone anchoring device of Fig. 1.
- Fig. 4A: shows a cross-sectional view of the receiving part according to the first embodiment taken along a line AA in Fig. 4B.
- Fig. 4B: shows a top view of the receiving part of Fig. 4A.
- Fig. 5A: shows a top perspective view of the receiving part shown in Fig. 4A.
- Fig. 5B: shows a bottom perspective view of the receiving part shown in Fig. 4A.
- Fig. 6A: shows a top perspective view of a locking element according to the first embodiment.
- Fig. 6B: shows a bottom perspective view of the locking element of Fig. 6A.
- Fig. 7A: shows a cross-sectional view of the locking element shown in Fig. 6A taken along a line BB in Fig. 7B.
- Fig. 7B: shows a top view of the locking element of Fig. 6A.
- Fig. 8A: shows a top perspective view of a retainer element according to the first embodiment.
- Fig. 8B: shows a bottom perspective view of the retainer element of Fig. 8A.
- Fig. 9A: shows a cross-sectional view of the retainer element of Fig. 8A taken along line DD in Fig. 9B.
- Fig. 9B: shows a top view of the retainer element of Fig. 8A.
- Fig. 10A: shows in a cross sectional view a first step of inserting a retainer element into a receiving part according to the first embodiment.
- Fig. 10B: shows in a cross sectional view a second step of inserting a retainer element into the receiving part according to the first embodiment.
- Fig. 10C: shows in a cross sectional view a third step of inserting a retainer element into the receiving part according to the first embodiment.
- Fig. 10D: shows in a cross sectional view a state in which the retainer element is brought and held in position by an engagement structure of the receiving part according to the first embodiment.
- Fig. 11A: shows in a perspective view a first step of inserting a locking element into the receiving part according to the first embodiment.
- Fig. 11B: shows in a perspective view a second step of inserting a locking element into the receiving part according to the first embodiment.
- Fig. 11C: shows in a perspective view a third step of inserting a locking element into the receiving part according to the first embodiment.
- Fig. 12A: shows in a cross sectional view a state of the coupling assembly wherein the locking element is in the first position according to the first embodiment.
- Fig. 12B: shows in a cross sectional view a state of the coupling assembly wherein a head of an anchoring element is inserted into the opening of coupling assembly according to the first embodiment.
- Fig. 12C: shows in a cross sectional view a state of the coupling assembly wherein the retainer element is expanded by the head of an anchoring element according to the first embodiment.
- Fig. 12D: shows in a cross sectional view a state of the coupling assembly wherein insertion of the head of the bone anchoring element is completed and the head is received by the hollow spherical segment-shaped recess portion of the retainer element, the locking element still being in the first position, according to the first embodiment.
- Fig. 13A: shows a step of locking the retainer element and simultaneously pre-locking the head of the anchoring element with friction fit according to the first embodiment.
- Fig. 13B: shows a step of inserting a rod into the device according to the first embodiment.
- Fig. 13C: shows a step of tightening a fixation element and final locking of the head of the anchoring element with selected orientation, with pressure forces between the parts being indicated, according to the first embodiment.
- Fig. 14: shows a perspective exploded view of a second embodiment of a polyaxial bone anchoring device.
- Fig. 15: shows a perspective view of the bone anchoring device of Fig. 14 in an assembled state.
- Fig. 16: shows a cross-sectional view of the bone anchoring device in an assembled state according to the second embodiment.
- Fig. 17: shows a perspective view of a bone anchoring element employed in conjunction with the second embodiment.
- Fig. 18A: shows a top perspective view of the locking element of Fig. 14 according to the second embodiment.
- Fig. 18B: shows a bottom perspective view of the locking element of Fig. 18A.
- Fig. 19A: shows a cross-sectional view of the locking element of Fig. 18A taken along a line EE in Fig. 19B.
- Fig. 19B: shows a top view of the locking element of Fig. 18A.
- Fig. 20A: shows in a cross sectional view a state of the coupling assembly wherein the locking element is in the first position according to the second embodiment.
- Fig. 20B: shows in a cross sectional view a state of the coupling assembly wherein insertion of the head of the bone anchoring element is completed and the head is received by the hollow spherical segment-shaped recess portion of the retainer element, the locking element still being in the first position, according to the second embodiment.
- Fig. 20C: shows a state, in which the retainer element is locked by the locking element such as to retain the head within the accommodation space according to the second embodiment.
- Fig. 20D: shows a step of tightening a fixation element and final locking of the head of the anchoring element with selected orientation, with pressure forces between the parts being indicated, according to the second embodiment.

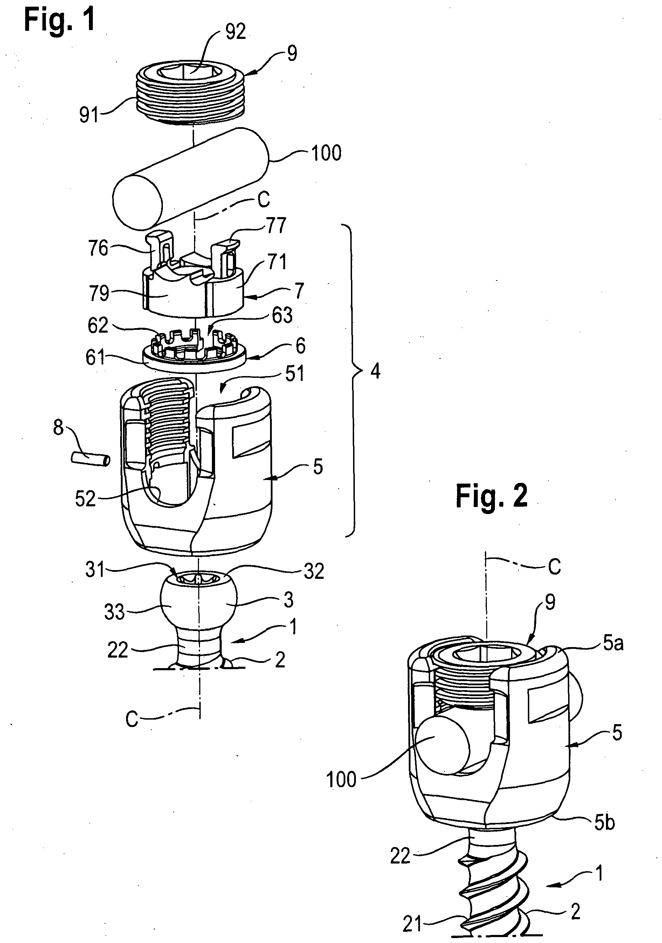

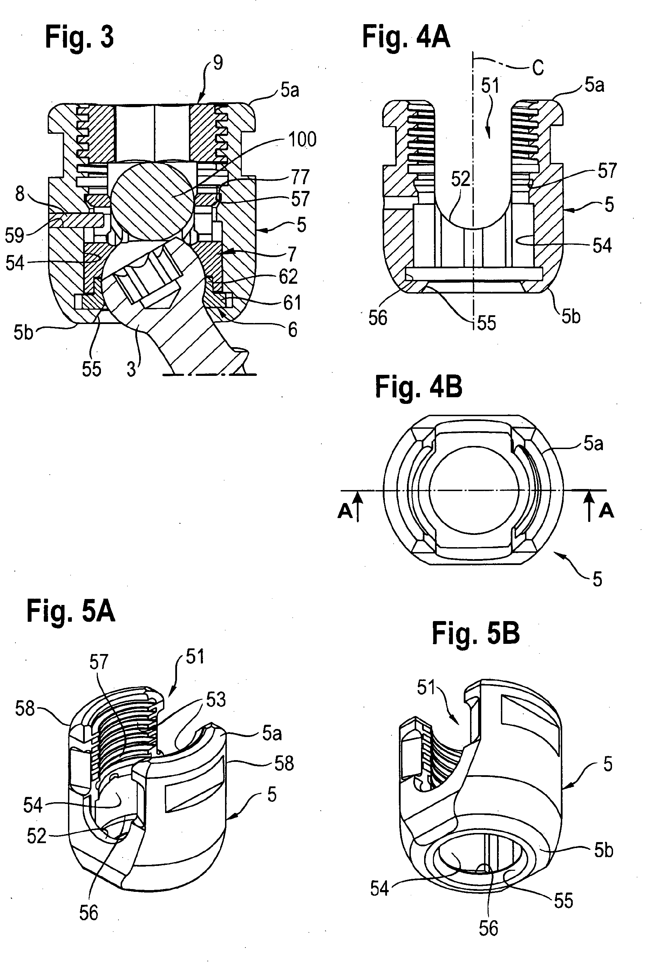

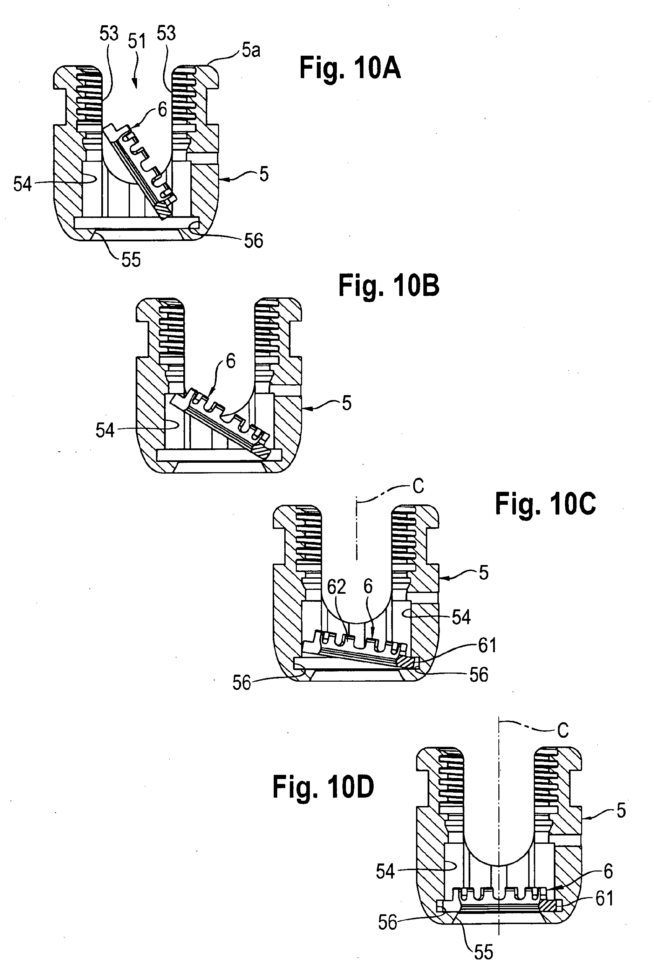

A first embodiment of the coupling assembly and the polyaxial bone anchoring device is explained with reference to Figs. 1-13C. Figs. 1-3 thereby provide an overview of the assembly and device, whereas Figs. 4A-9B provide details of the more relevant parts. Figs. 10A-13C illustrate the use of the assembly and device according to the first embodiment.

As shown in Fig. 1, a polyaxial bone anchoring device comprises an anchoring element 1, a coupling assembly 4 and a fixation element 9, such as a set screw, for connecting the rod 100 with the anchoring element 1. The anchoring element 1 comprises a spherically segment-shaped head 3 having a flat top portion 32, an engagement portion 31 for engagement by an external tool such as a driver, and a spherical portion 33. The anchoring element 1 further has a shank 2 provided with a bone thread 21 and a neck portion 22 connecting the shank to the head 3.

The coupling assembly 4 comprises a receiving part 5, a retainer element 6, a locking element 7 and a pin 8. The receiving part 5 is of a substantially cylindrical shape with optionally flat side and engagement portions for engagement by an external tool. The receiving part 5 has a top end 5a and a bottom end 5b and an inner bore 51 extending from the top end 5a in a direction towards the bottom end 5b. As can be seen in more detail in Figs. 3-5B, an opening 55 is formed at the bottom end 5b that permits introduction of a head 3 into an accommodation space 54, which extends in the receiving part from the opening 55 towards the bore 51.

The accommodation space 54 is in this embodiment of cylindrical shape and has at its bottom end adjacent to the opening 55 an annularly extending groove functioning as engagement structure 56 for the retainer element 6 and arranged to receive an annularly shaped engagement portion of the retainer element 6 described below. Each, the bore 51, the accommodation space 54 and the annular engagement structure 56 are symmetric and coaxial with respect to a central axis C of the receiving part 5.

A U-shaped recess 52 extends from the top end 5a of the receiving part 5 defining two free legs 58 at the inner side of which are provided threads 53. A fixation element 9 having an engagement portion 92 for an external tool such as a driver (not shown) and an external thread 91 is provided to be threaded into threads 53 of receiving part 5 such as two tighten, fixate and lock a rod 100 which inserted into a recess 52 as well as head 3 which is inserted into the accommodation space 54.

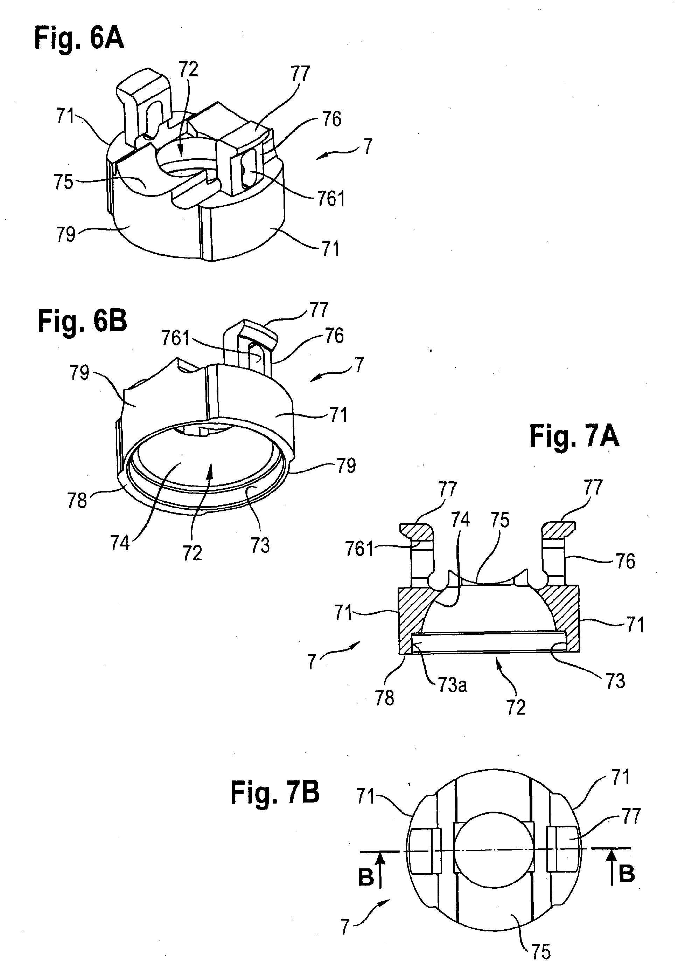

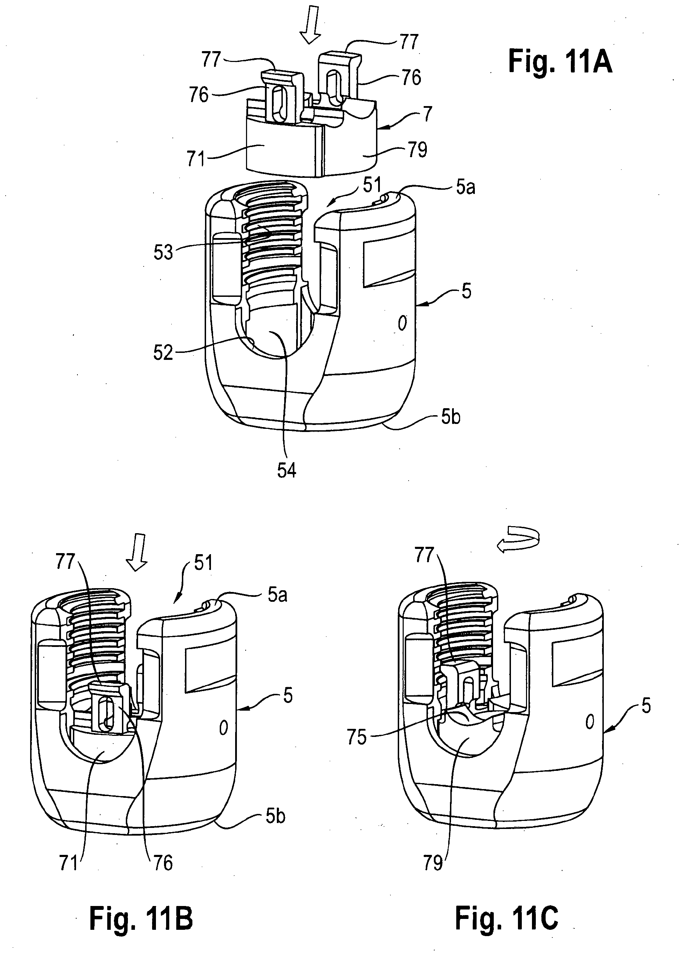

The locking element 7 is explained in more detail with reference to Figs. 6A-7B. Locking element 7 has two outer cylindrical surface portions 71 arranged opposite with respect to each other. Both portions 71 reveal an outer diameter of the locking element 7 substantially corresponding to an inner diameter of the accommodation space 54. Consequently, when locking element 7 is provided to the accommodation space 54, locking element 7 is in sliding engagement with the inner walls of the accommodation space 54, thereby being translational movable along the central axis C. Further, the circumference of locking element 7 comprises two opposite recessed outer cylindrical surface portions 79 revealing a reduced diameter of the locking element in a transverse direction. Both portions 79 extend respectively between the outer cylindrical surface portions 71. The reduced diameter of recessed outer cylindrical surface portions 79 is selected to be less than an inner diameter of the inner bore 51, in particular less than an inner diameter of the threads 53 to allow an introduction of the locking element 7 from the top end 5a in a posture rotated by 90 degrees as compared with the finally assembled state, see Figs. 11A-C.

Locking element 7 further has on its top face a rod-receiving portion 75, which is arranged to receive rod 100 as shown in Figs. 1 and 2. Further, an inner coaxial bore 72 provides access to an inner cavity 72 of the locking element 7, which comprises in an upper portion a spherical segment-shaped recess 74 for receiving the partially spherical head 3, and in a bottom portion a recess 73 having a substantially cylindrical inner wall 73a. Inner wall 73a may also have a conical or otherwise tapered shape. Recess 73 is adapted to receive a corresponding cylindrical, conical or tapered, etc. engagement portion 62 ("second engagement portion") of the retainer element 6 to be described below.

The spherical segment-shaped recess 74 of the locking element 7 is sized and adapted to the spherical portion 33 of head 3 such as to allow selecting a specific polyaxial orientation of the anchoring element 1 in a pre-locked or finally locked state described below according to the needs. To establish a pre-locked state in this embodiment, two arms 76 are provided in a top portion of locking element 7, whose ends are provided with engagement shoulders 77, which may resiliently engage with corresponding engagement shoulders 57 provided in a wall portion of the inner bore 51, for example near the bottom end of threads 53. The length of the arms 76 and the vertical height positions of engagement shoulders 57 at the inner wall of the bore 51 are selected such that when shoulders 57, 77 snap-in and mutually engage with each other, the second engagement portion 62 of retainer element 6 is at least partially received in recess 73 having the inner wall 73a. In such a state, the retainer element 6 is locked and release of the head 3 from the retainer element 6 is prevented, which is denoted herein as a pre-locked state.

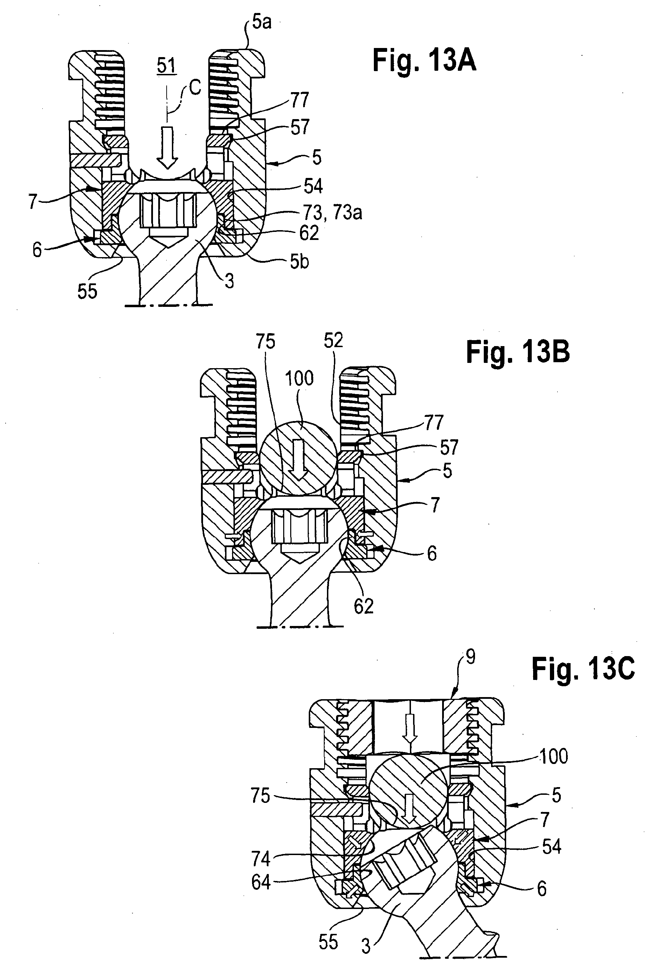

As can be seen from Figs. 3 and 13A, when a rod 100 is received by the rod-receiving portion 75 of locking element 7, further exertion of a pressure force on rod 100 by a fixation element 9 will adversely lead to a transfer of the pressure force onto the locking element 7, which itself transfers the pressure force via its inner spherical segment-shaped recess onto the head 3 of anchoring element 1. As such, the locking element 7 of the first embodiment functions as the pressure element.

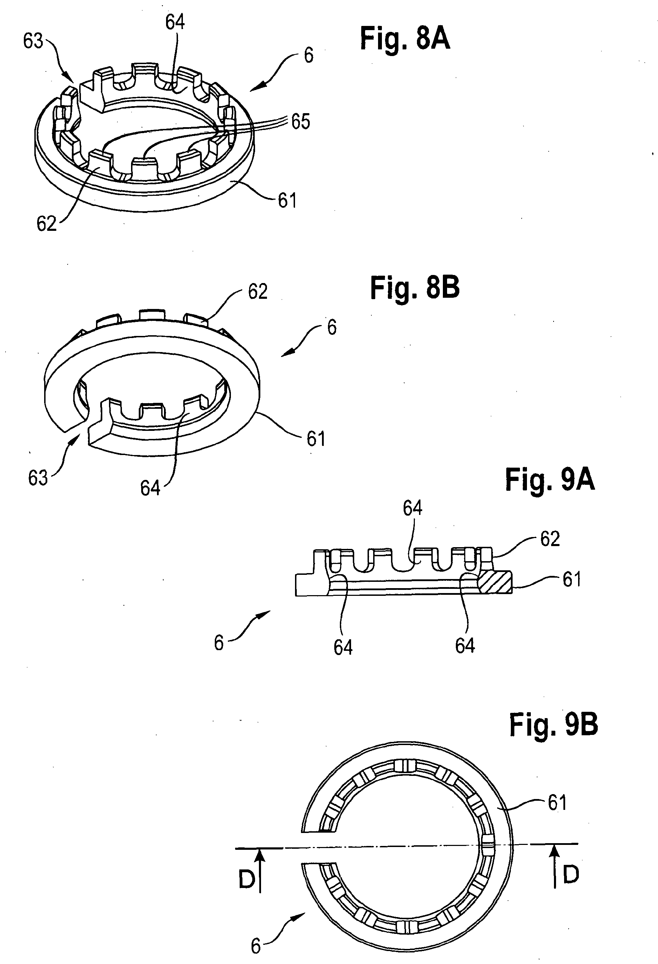

Details of the retainer element 6 are explained with reference to Figs. 8A-9B. The retainer element 6 substantially has a ring-shape with a slit 63. Retainer element 6 thus represents an open ring. Slit 63 allows the retainer element 6 to be radially expanded and/or compressed. Retainer element 6 comprises a ("first") substantially annular engagement portion 61, which is configured to be received by the annular engagement groove or engagement structure 56 provided at the accommodation space 54, and the second engagement portion 62, which in this embodiment has a slightly conical outer appearance, but may also be cylindrical or otherwise tapered. The second engagement portion 62 has a plurality of regularly arranged flanges or lugs 65 separated by U-shaped slots to yield a crown-like arrangement. The invention is not limited to the specific arrangement shown herein. Other kinds of projections or a single conical, cylindrical, rounded or tapered wall is possible as well. The second engagement portion 62 is configured to be received by the recess 73, which may have a cylindrical, conical, tapered or rounded inner wall 73a, of locking element 7. Once received in the recess 73 of locking element 7, the engagement portion 62 is prevented from further expansion when it abuts on inner wall 73a. In this embodiment, the second engagement portion 62 extends from the first engagement portion 61 towards the top end 5a of the receiving part 5, when being inserted therein. The first and second engagement portions 61, 62 are formed as a monolithic, single piece.

While the outer appearance of the second engagement portion 62 may be cylindrical, conical, tapered or rounded etc., an inner wall of the retainer element 6 includes a hollow spherical segment-shaped portion 64 as can particularly be seen in Fig. 9A, wherein its curvature corresponds to that of spherical portion 33 of head 3 of anchoring element 1. In other words, spherical segment-shaped portion 64 is arranged to receive and retain the head 3 of anchoring element 1.

In order to retain the head 3 of anchoring element 1 in a locked state of the retainer element 6, the inner diameter of the retainer element 6 in a state, when the second engagement portion 62 is received in recess 73 of the locking element 7 (which may also be a compressed state of the retainer element 6) is less than that of the head 3 to prevent release of the head from retainer element 6.

The assembly and use of the coupling assembly 4 according to the first embodiment is explained in what follows with reference to Figs. 10A-13C.

The insertion of the retainer element 6 into the receiving part is explained with reference to Figs. 10A-10D. As can be seen in Fig. 10A, the retainer element 6 is inserted into the bore 51 from a top end 5a of receiving part 5. Since an outer diameter of retainer element 6 is larger than an inner diameter of the bore 51, in particular of inner threads 53, retainer element 6 is inserted in an inclined fashion and then rotated towards a horizontal posture when it reaxches its predetermined position in the accommodation space 54. As can be seen in Figs. 10B and 10C, once the retainer element 6 has reached the accommodation space 54 having a larger inner diameter as compared with bore 51, the retainer element 6 can be brought into a position less inclined and closer to a coaxial orientation with respect to central axis C.

In an unstressed, unbiased state of the retainer element 6 an outer diameter of annular engagement portion 61 is larger than an inner diameter of accommodation space 54 such that the retainer element 6 abuts on an inner wall of accommodation space 54. The larger diameter of retainer element 6 is selected to permit the retainer element 6 being held in position coaxial with central axis C when the annular engagement portion 61 enters and snaps into the annular engagement groove or engagement structure 56 of receiving part 5, as it is depicted in Fig. 10D. In this step shown, retainer element 6 is slightly compressed from the unstressed state until it snaps into the groove, whereafter it expands again into the unstressed state. The groove of engagement structure 56 has a larger inner diameter than the outer diameter of the annular engagement portion 61 of retainer element 6 in order to provide enough tolerance, or play, to allow expansion of the retainer element 6 from the unstressed state, when the head 3 of anchoring element 1 is inserted as described below.

Insertion of the locking element 7 into the receiving part 5 is explained with reference to Figs. 11A-11C. As shown in Fig. 11A, the locking element 7 is introduced into the bore 51 of receiving part 5 from the top end 5a. Thereby, the protruding outer cylindrical surface portions 71 face towards the U-shaped recesses 52, i.e., the orientation of the locking element 7 with respect to its final installed state is offset by 90 degrees. Further, recessed outer cylindrical surface portions 79 face the threads 53 to allow safe passage of bore 51.

As shown in Fig. 11B, when the locking element 7 (more specifically, its bottom or base portion including surfaces 71, 79) has passed the inner threads 53 and reached the accommodation space 54 of receiving part 5, the locking element 7 may be rotated by 90 degrees by sliding engagement between the outer cylindrical surface portions 71 with the inner wall portions of accommodation space 54.

As shown in Fig. 11C, locking element 7 is then rotated into its final position by 90 degrees with arms 76 and shoulders 77 oriented towards the legs 58 having the threads 53 and shoulders 57, such as to be prepared for engagement of shoulders 57, 77 to establich the pre-locked state explained below with respect to Fig. 13A. In this state, the protrusions of the arms 76 which bear the shoulders 77, rest upon corresponding protrusions at the inner wall of the bore 71 which provide those shoulders 57 facing down towards the opening 55 and bottom end 5b. In other words, shoulders 57, 77 are not yet engaged.

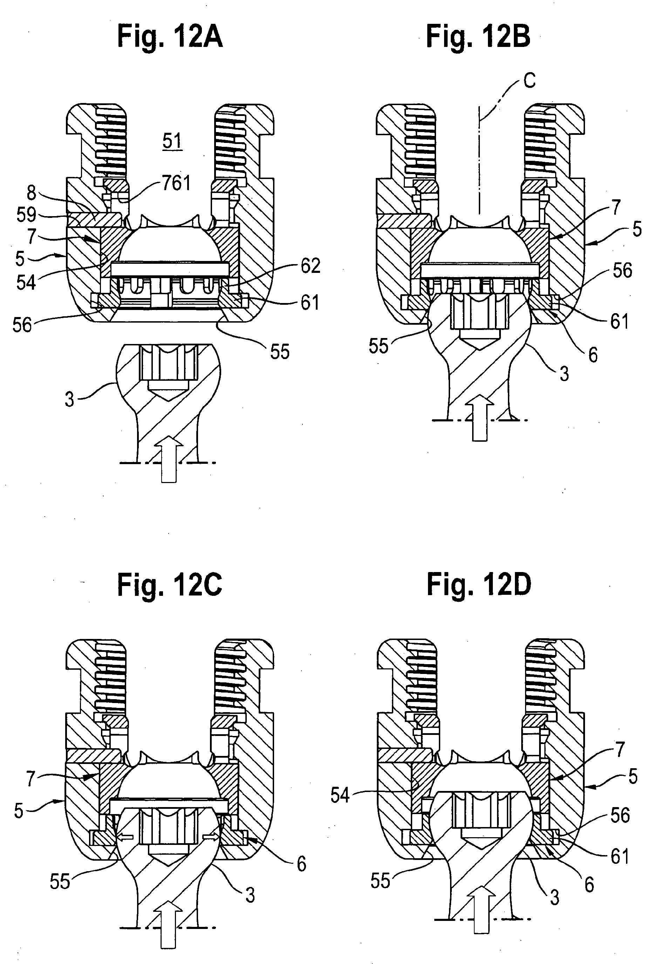

Figs. 12A-12D detail the process of inserting the head 3 of anchoring element 1 in the accommodation space 54. As shown in Fig. 12A, prior to insertion of head 3 a pin 8 is inserted into an elongate hole 59 which is formed in a side wall of the receiving part 5, such that a tip portion thereof protrudes into the bore 51 of receiving part 5 and into an elongated aperture 761 which is formed in arms 76 of locking element 7. In this state, locking element 7 is prevented from unintended rotation back into that positon (by 90 degrees), in which it has previously been inserted..

Next, as shown in Fig. 12B, head 3 of anchoring element 1 is introduced into opening 55 whereby surface portion 33 contacts an inner wall of retainer element 6. Since flange-like annular engagement portion 61 is firmly received in the engagement groove of engagement structure 56 of receiving part 5, the retainer element 6 is held in position coaxial with central axis C wherein no translation along central axis C is possible, and retainer element 6 starts to expand in a radial direction responsive to further insertion of the head which locally increases in diameter as shown by the arrows in Fig. 12C. The radial play or tolerance in engagement groove 56 is thereby the same as or even larger than a difference between the maximum outer diameter of head 3 and minimum inner diameter of retainer element 6, i.e., equal to or larger than the amount of expansion.

As shown in Fig. 12D, further insertion of head 3 allows retainer element 6 to return into an unstressed or at least less expanded state. The retainer element 6 along with receiving part 5 snaps onto head 3 by virtue of its compressive force. Fig. 12D still shows the unlocked "first" position of the locking element 7 with respect to retainer element 6. Nevertheless, since spherical head 3 is now received in mating inner spherical segment-shaped surface 64 of retainer element 6 in this state, a sufficient amount of friction between is generated therebetween to maintain an angular orientation of anchoring element 1 as desired with respect to receiving part 5 during assembly and during in-situ applications prior to final locking. As a consequence, a friction-fit connection between head 3 and retainer element 6 is achieved.

Steps of pre-locking and final locking of the head 3 of anchoring element 1 as well as locking of retainer element 6 is explained with reference to Figures 13A-13C. As shown in Fig. 13A, locking of retainer element 6 and pre-locking of head 3 of anchoring element 1 is accomplished by moving the locking element 7 from the first position (shown in Fig. 12D) to a second position, in which second engagement portion 62 is received in recess 73 or cavity 72 of locking element 7, such that engagement portion 62 is a radially engaged from outside and hindered from further expansion in this embodiment. As noted above, even some play between inner wall 73a and engagement portion 62 would be acceptable as long as the head 3 is prevented from being released. Retainer element 6 is thus locked and head 3 cannot be released in this state any more from accommodation space 54, or opening 55.

In a next step shown in Fig. 13B, a rod 100 is inserted into the U-shaped recess 52 and received by rod receiving surface 75 of locking element 7. At this instance, a compressive force exerted by locking element 7 onto the second engagement portion 62 of retainer element 6 is only exerted when forces trying to remove the anchoring element 1 are generated.

Figures 13A und 13B also show a pre-locked state of head 3 in the receiving part. In conjunction with the locking of retainer element 6 due to the sliding movement of locking element 7 towards the opening 55 and bottom end 5b along the central axis C, arms 76 of locking element 7 flex inwards and shoulders 77 provided at the tips of arms 76 of locking element 7 eventually latch resiliently into corresponding recesses providing shoulders 57 at the inner wall of inner bore 51 of receiving part 5. In this state, see Fig. 13A, the retainer element 6 is prevented from expansion beyond inner walls 73a such that the head 3 cannot be released any more from the retainer element 6 ("pre-lock"). At the same time, the above described friction-fit connection between the head 3 and the retainer element 6 is maintained.

Fig. 13C shows the steps of final locking of head 3 in receiving part 5. Fixation element 9 is screwed into thread 53 of receiving part 5 exerting a downward force onto rod 100. Rod 100 transfers this pressure onto rod receiving portion 75 of locking element 7 which adversely exerts a pressure force on head 3 thereby pressing head 3 against hollow spherical segment-shaped portion 64 of retainer element 6. Retainer element 6 cannot expand since it is locked by locking element 7, and retainer element 6 cannot slide down towards the opening 55 and bottom end 5b, since its engagement portion 61 is received in engagement groove 56. The direction of pressure forces exerted as indicated by arrows in Fig. 13C.

Since the retainer element 6 is dimensioned and shaped to allow insertion from a top end 5a as shown in Figures 10A-10D, the bottom opening 55 of receiving part 5 may have a smaller diameter, which further supports the retainer element 6 in engagement groove 56 adjacent opening 55. This is made possible by exerting pressure forces directly onto the head 3 via the locking element 7 instead of the retainer element 6, as would be suggested by the spring chuck in above described document US 6,248,105 B1. As compared with that spring chuck, the retainer element 6 is thus reduced in function to retaining the head 3, such that its dimensions along the central axis C (i.e., its height) may be selected smaller allowing introduction from the top end 5a, which adversely permits to select a smaller width for the bottom opening 55.

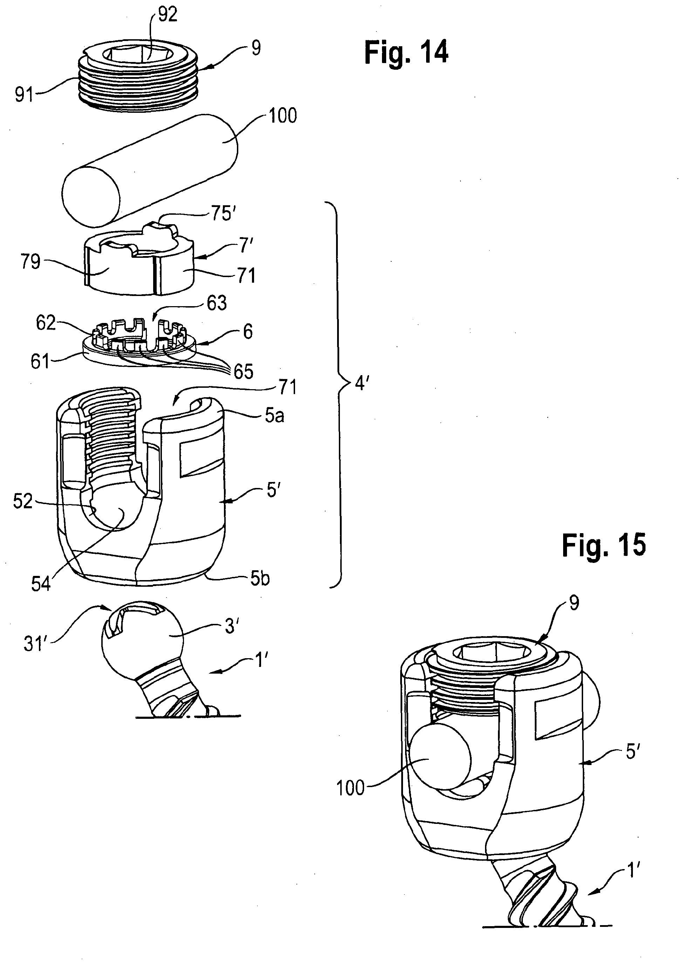

A second embodiment of the present invention is explained with respect to Figures 14-20D. Like parts as in the first embodiment are referenced with the same numerals, and description thereof will not be repeated herein. The second embodiment differs from the first embodiment mainly with regard to the locking element 7' and the anchoring element 1', and to some extent with respect to the receiving part 5'. More specifically, the second embodiment does not provide for a pre-locked state, and the locking element 7' is not arranged as a pressure element. However, features of the retainer element 6 and the interaction with the locking element 7' as well as with the engagement structure are the same between both embodiments.

As becomes apparent from Figures 14-16, the coupling assembly 4' comprises a receiving part 5', retainer element 6,and locking element 7'. The receiving part 5' differs from the receiving 5 of the first embodiment in that no bore hole 59 is provided, which would allow introducing a pin 8 to lock the locking element 7 in a pre-lock position. Instead, a crimp bore 591 is formed in a side wall of receiving part 5'.

The locking element 7' of the second embodiment differs from the locking element 7 of the first embodiment in that no arms 76 or shoulders 77 are provided which in the first embodiment facilitate a pre-locked state with friction fit between the locking element (pressure element) and the head 3. Rather, the locking element 7' of the present embodiment solely serves to lock the retainer element 6 to prevent release of the head 3' from the receiving part 5'. Nevertheless, a small recess 711 is formed in the outer cylindrical surface 71 of locking element 7', which is configured to receive a deformable portion at the end wall of crimp bore 591, when this portion is deformed to project into the accommodation space 54 using an external tool (not shown).

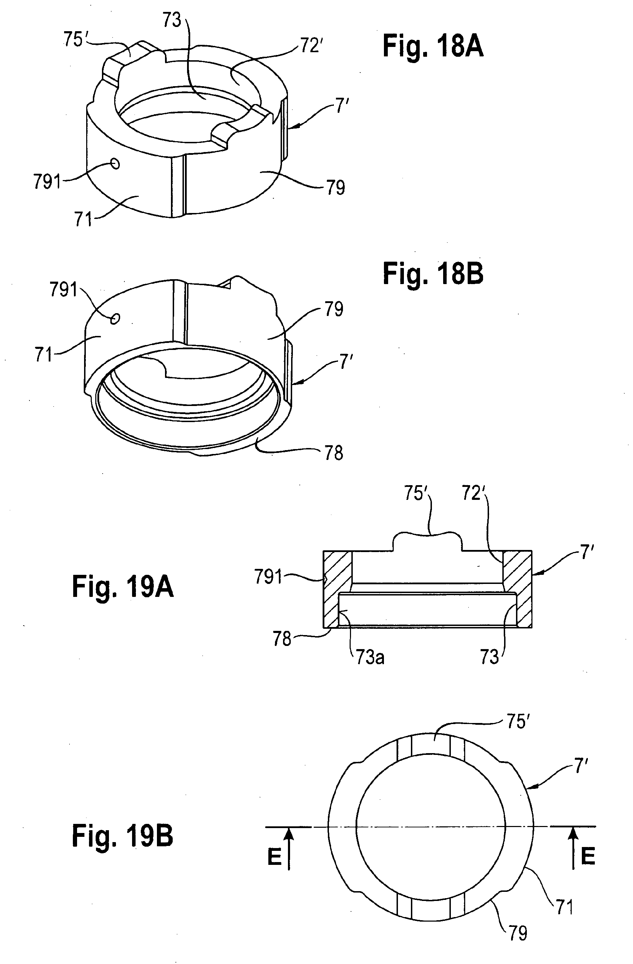

As shown in Fig. 18A-19B, the locking element 7' has a substantially cylindrical shape with outer cylindrical surface portions 71 and outer recessed cylindrical surface portions 79 like in the first embodiment. However, locking element 7' does not have an inner spherical segment-shaped recess. Rather, locking element 7' has an inner bore 72' allowing access to an engagement portion 31' of anchoring element 1', and the recess 73 having inner wall 73a as in the first embodiment, which functions to engage the second engagement portion 62 of retainer element 6 to lock expansion of the retainer element 6. Recess 73 is dimensioned and shaped similar to the first embodiment.

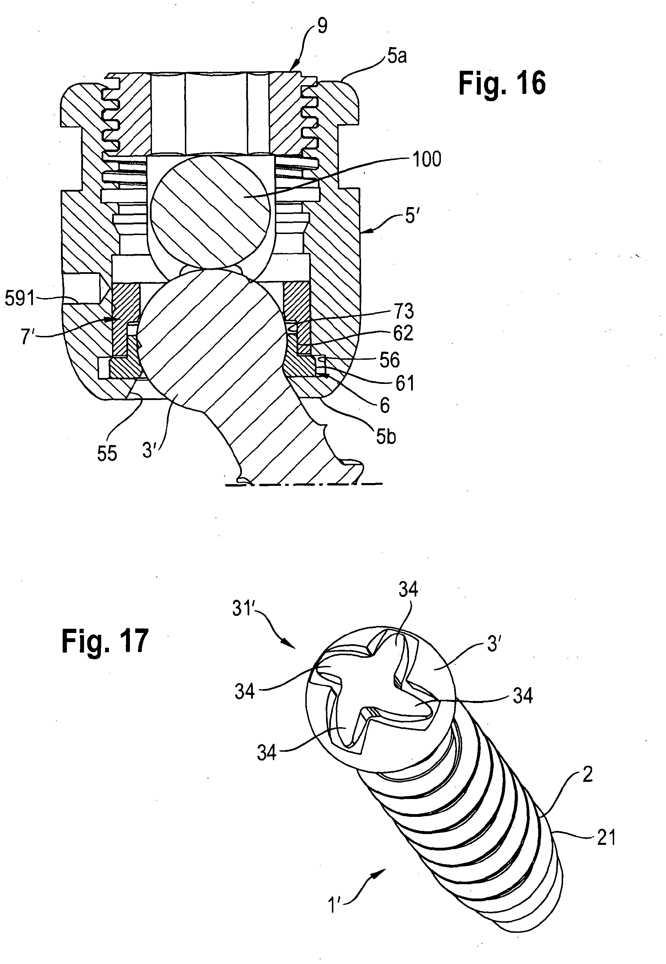

With this structure, locking element 7' of the second embodiment does not interact with the head 3' of anchoring element 1, and as can be seen from Fig. 16, the rod 100 directly exerts a pressure force onto head 3' of the anchoring element 1'.

The anchoring element 1' according to the second embodiment is shown in Fig. 17. The bone anchoring element 1' comprises a spherical head 3' that has a spherical outer surface even at the free upper end. The head as a whole is spherical. An engagement portion 31' provided at the free end comprises wings that extend in a spiral-like manner from a center point of the upper free end. In this embodiment, the engagement portion comprises four wings. The four wings 34 describe an outer contour of a cross with arms each bent in the same direction. By means of this, the engagement surface of a driver is enhanced compared to usual polygon or other recesses. The loads that can be transferred are higher. Engagement portions for drivers and corresponding tools with a similar shape are known under the trademark Mortorq®. Due to the spherical contour of the head, the head 3' may extend through the recess 73 and inner bore 72' such that a direct pressure transfer between the rod 100 and the head 3' can be established.

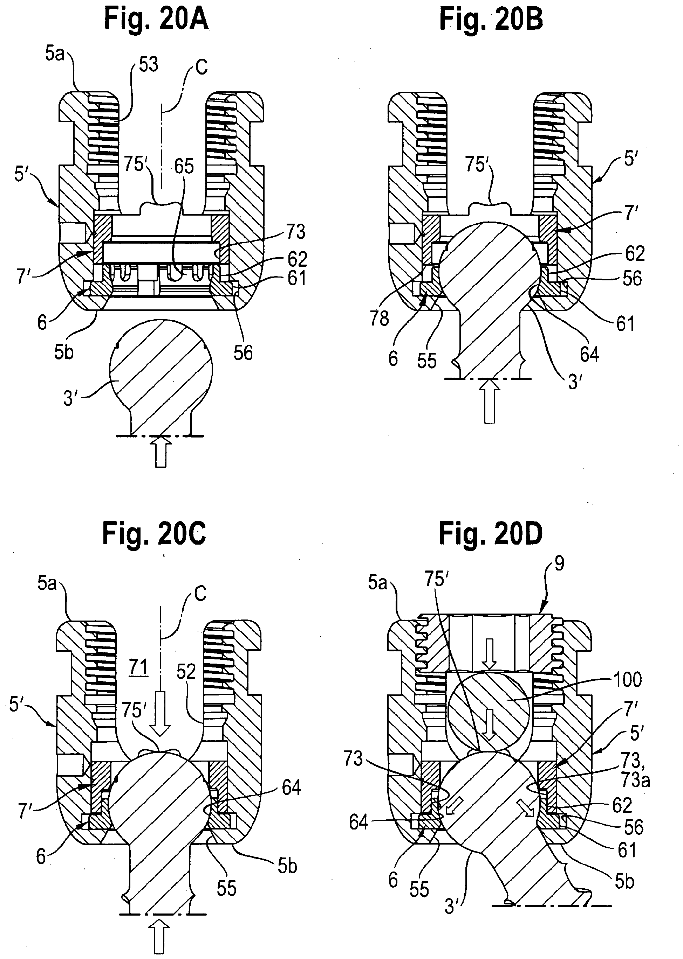

Assembly and use of the coupling assembly 4' and polyaxial bone anchoring device comprising the same according to the second embodiment is explained with respect to Figs. 20A-D.

As shown in Fig. 20A, which corresponds to the state depicted in Fig. 12A regarding the first embodiment, prior to insertion of head 3' locking element 7' is introduced into bore 71 of receiving part 5' similar as shown in Figs. 11A-C.. Thereby, rod receiving surface 75' is aligned with the rod receiving channel or U-shaped recess 52 of receiving part 5'. The retainer element 6 is already held in position by the groove of engagement structure 56 which received the first engagement portion 61 of retainer element 6.

Next., as shown in Fig. 20B, which correpsonds to the situation depicted in Fig. 12D with regard to the first embodiment, the head 3' has been inserted into the bottom opening 55 of receiving part 5' and received by the inner hollow spherical segment-shaped portion 64 of retainer element 6. In Figs. 20A and 20B, the locking element 7' is in the first unlocked position. Locking element 7' may be held in this temporary position by deforming a portion at an end wall of crimp bore 591, which portion then projects from an inner wall of accommodation space 54 and into small recess 711 of locking element 7'.

As shown in Fig. 20C, which corresponds to the situation depicted in Fig. 13A with regard to the first embodiment, the locking element 7' is moved along the central axis C in sliding engagment between surfaces 71 and and an inner wall of the accommodation space 54 towards the opening 55 and bottom end 5b, wherein the inner wall 73a of cavity 73 is guided onto the second engagement portion 62 of retainer element 6 such as to lock the same. The (comparatively small) force exerted to move the locking element 7' may be achieved manually, by a tool, or by already inserting the rod 100 with small pressure force. As in the first embodiment, the locking element 7' hinders further expansion of retainer element 6, when the second engagement portion abuts on inner wall 73a of locking element 7'.

It may be noted that since there is no spherical segment-shaped recess in locking element 7', inner bore 72' may be shaped such that there is no or almost no contact between locking element 7' and head 3' of anchoring element 1', when head 3' is received in hollow spherical segment-shaped recess 64 of retainer element 6.

As shown in Fig. 20D, which corresponds to the situation depicted in Fig. 13C with regard to the first embodiment, final locking is accomplished by attaching and tightening fixation element 9 at the top end 5a of receiving part 5', thereby exerting a pressure force onto rod 100 received in rod receiving portion 75' of locking element 7'. Rod 100 transfers the pressure force onto the spherical head 3'. Spherical head 3' is adversely pressed against retainer element 6, which may neither expand nor release head 3', since its second engagement portion 62 is radially bound by inner wall 73a of cavity 73, nor may axially move further towards opening 55 since its first engagement portion 61 is received in the annular groove of engagement structure 56.

The bone anchoring device of the first and second embodiments as a whole or in parts may be made of a bio-compatible material, such as a bio-compatible metal or a metal alloy, for example titanium, stainless steel, a nickel-titanium alloy, for example nitinol, or of bio-compatible plastic materials, such as, for example, polyetheretherketon (PEEK) or of a bio-compatible ceramic material. In particular, it may be contemplated that the retainer and/or locking elements are made of a superelastic nickel-titanium alloy or of beta titanium.

Further modifications of the coupling assembly and polyaxial bone anchoring device may be contemplated.

Other possible modifications of the receiving part may include, for example, instead of the U-shaped recess being perpendicular to the central axis, a recess for the rod may be inclined, open to the side, or in the form of a closed channel. Other kinds of locking devices including outer nuts, outer caps, bayonet locking devices, or others are also possible as noted above. In particular, a two-part locking device that includes a first locking element that exerts pressure via the pressure element onto the head and the second locking element that exerts pressure only onto the rod to lock the head and the rod independently, may also be used. In some embodiments, the inner surface portion of the locking element that contacts the head (first embodiment), may not necessarily be spherically-shaped. The inner surface portion may have any other shape that is suitable to exert pressure onto the head. Also, the design of the locking element can be different and is not limited to the specific design shown in the first or second embodiments.

Instead of the pin for retaining the pressure element and for aligning the pressure element with respect to the channel for receiving the rod of the receiving part, other retaining mechanisms can be used.

In the above embodiments, the accommodation space is of a substantially cylindrical shape. However, other shapes or deviations from a cylindrical may be possible as well which allow a locking element (having corresponding shapes or deviations) to move between the respective first and second positions connected with the unlocked and locked states.

In the above embodiments, the locking element is shown either as being provided with arms and shoulders to effect a pre-locked state or without arms. However, both embodiments may also be formed without, or the other way around with arms, and with corresponding functions, respectively.
The coupling assembly of the above or further embodiments including the retainer and the locking element (be it embodied as a pressure element or not) may be in situ snapped-on to the head 3, 3' of anchoring element 1, 1' when the anchoring element is inserted into a bone, e.g., a vertebra, or in a not yet implanted state.

## Claims

1. A polyaxial bone anchoring device comprising:
a bone anchoring element (1) having shank (2) for anchoring to bone and a head (3), and a coupling assembly for coupling a rod to the bone anchoring element, the coupling assembly (4) comprising:
a receiving part (5) having a first end (5a), a second end (5b), a central axis (C) extending through the first end (5a) and second end (5b), an accommodation space (54) for accommodating the head (3) of an anchoring element (1), the accommodation space having an opening (55) at the second end (5b) sized so as to permit the insertion of the head (3) and a bore (51) extending from the accommodation space to the first end (5a), and a recess (52) for receiving a rod (100),
a retainer element (6) configured to be positioned at least partially in the accommodation space (54) and being radially expandable and/or compressible so as to allow retaining the head (3) which is inserted through the opening (55);
wherein the retainer element (6) is held in position adjacent the opening (55) by a first engagement portion (61) which engages an engagement structure (56) provided at or in the accommodation space (54) of the receiving part (5);
a locking element (7), which is configured to be arranged at least partially in the accommodation space (54), the locking element (7) being movable from a first position, in which the retainer element (6) held in position adjacent the opening (55) by the engagement structure (56) is allowed to expand and release the inserted head (3), to a second position, in which radial expansion of the retainer element (6) held in position adjacent the opening (55) by the engagement structure (56) is hindered so as to prevent release of the inserted head (3);
wherein the retainer element (6) further includes a second engagement portion (62) for engagement by the locking element (7) when moved in to the second position, such as to prevent the retainer element (6) from expansion;
wherein, when the locking element (7) is in the second position, the head (3) received in the accommodation space (54) can be locked, wherein the locking element (7) exerts a pressure force directly onto the head (3) to press the head (3) against the retainer element (6).

2. A polyaxial bone anchoring device comprising:
a bone anchoring element (1') having shank (2) for anchoring to bone and a head (3'), a rod (100), and a coupling assembly for coupling a rod to the bone anchoring element, the coupling assembly (4') comprising:
a receiving part (5') having a first end (5a), a second end (5b), a central axis (C) extending through the first end (5a) and second end (5b), an accommodation space (54) for accommodating a head (3') of an anchoring element (1'), the accommodation space having an opening (55) at the second end (5b) sized so as to permit the insertion of the head (3') and a bore (51) extending from the accommodation space to the first end (5a), and a recess (52) for receiving a rod (100),
a retainer element (6) configured to be positioned at least partially in the accommodation space (54) and being radially expandable and/or compressible so as to allow retaining the head (3') which is inserted through the opening (55);
wherein the retainer element (6) is held in position adjacent the opening (55) by a first engagement portion (61) which engages an engagement structure (56) provided at or in the accommodation space (54) of the receiving part (5');
a locking element (7'), which is configured to be arranged at least partially in the accommodation space (54), the locking element (7, 7') being movable from a first position, in which the retainer element (6) held in position adjacent the opening (55) by the engagement structure (56) is allowed to expand and release the inserted head (3'), to a second position, in which radial expansion of the retainer element (6) held in position adjacent the opening (55) by the engagement structure (56) is hindered so as to prevent release of the inserted head (3');
wherein the retainer element (6) further includes a second engagement portion (62) for engagement by the locking element (7') when moved in to the second position, such as to prevent the retainer element (6) from expansion;
wherein, when the locking element (7') is in the second position, the head (3') received in the accommodation space (54) can be locked, wherein the rod (100) exerts a pressure force directly onto the head (3') to press the head (3') against the retainer element (6).

3. The polyaxial bone anchoring device of claim 1 or 2, wherein the retainer element (6) defines an open or closed ring-shape.

4. The polyaxial bone anchoring device of one of claims 1 to 3, wherein the retainer element (6) includes one or more slits (63) in order to allow the retainer element (6) to be expanded and/or compressed.

5. The polyaxial bone anchoring device of one of claims 1 to 4, wherein the first engagement portion (62) of the retainer element (6) has an annular shape or annular segment shape.

6. The polyaxial bone anchoring device of one of claims 1 to 5, wherein the engagement structure (56) provided at or in the accommodation space (54) is a recess formed at an inner wall of the accommodation space (54).

7. The polyaxial bone anchoring device of claim 6, wherein the recess has a shape of an annular groove.

8. The polyaxial bone anchoring device of one of claims 1 to 7, wherein the second engagemenmt portion of the retainer element (6) has a cylindrical or conical shape which is coaxial with the central axis (C) when the retainer element (6) is held in position in the accommodation space (54).

9. The polyaxial bone anchoring device of one of claims 1 to 8, wherein the retainer element (6) further includes: a hollow spherical segment-shaped portion (64) configured to receive the head (3, 3') therein.

10. The polyaxial bone anchoring device of one of claims 1 to 8, wherein the locking element (7, 7') comprises:
an outer at least partially cylindrical surface (71) dimensioned to be in sliding engagement with an inner wall of the accommodation space (54) when being received therein at least between the first and second positions; and
an inner cavity (72) including a recess (73) having an inner wall (74) configured to engage the second engagement portion (62) of the retainer element (6), which prevents the retainer element (6) from expansion when the locking element (7, 7') is in the seond position.

11. The coupling assembly of of claim 10, wherein the locking element (7) is a pressure element which is arranged to receive and transfer a pressure force from a fixation element (9), which is attached to the bore (51) of the receiving part (5) at the first end (5a), and/or a rod (100), which is received in the recess (52) of the receiving part (5), towards the head (3) in order to tight lock the head (3).

12. The polyaxial bone anchoring device of claim 11, wherein the inner cavity (72) of the locking element (7) further comprises an inner hollow spherical segment-shaped recess (74) configured to receive the head (3, 3').

13. The polyaxial bone anchoring device of one of claims 1 to 12, wherein the locking element (7, 7') comprises a rod receiving portion (75) configured to receive the rod (100).

14. The polyaxial bone anchoring device of one of claims 1 to 12, wherein the locking element (7, 7') comprises an first engagement shoulder (77) configured to engage a second engagement shoulder (57) provided in the bore (51) or accommodation space (54), when the locking element (7, 7') is in the second position.

15. The polyaxial bone anchoring device of one of claims 1 to 14, wherein the retainer element (6) and/or the locking element (7, 7') are configured such as to be inserteable into the bore (51) and accommodation space (54) of the receiving part (5, 5') from the first end (5a).

16. The polyaxial bone anchoring device of claim 2, wherein
the head (3') of the bone anchoring element comprises a spherical end surface with an engagement recess (31') for a driver wherein the recess comprises a plurality of wing portions (34) that extend in a spiral-like manner from a center point of the free end surface; and
the retainer element (6') and the locking element (7') are shaped such that the head (3') can partially extend therethrough in order to be directly engaged by the inserted rod (100).

## Patentansprüche

1. Eine polyaxiale Knochenverankerungsvorrichtung, umfassend:
ein Knochenverankerungselement (1) mit einem Schaft (2) zum Verankern in einem Knochen und einem Kopf (3), und eine Kopplungsanordnung zum Koppeln eines Stabs mit dem Knochenverankerungselement, wobei die Kopplungsanordnung (4) umfasst:
ein Aufnahmeteil (5) mit einem ersten Ende (5a), einem zweiten Ende (5b), einer sich durch das erste Ende (5a) und das zweite Ende (5b) erstreckenden Mittenachse (C), einem Aufnahmeraum (54) zum Aufnehmen des Kopfes (3) eines Verankerungselements (1), wobei der Aufnahmeraum eine Öffnung (55) an dem zweiten Ende (5b) besitzt, der so bemessen ist, dass er das Einsetzen des Kopfes (3) erlaubt, und einer sich von dem Aufnahmeraum zu dem ersten Ende (5a) erstreckenden Bohrung (51), und einer Ausnehmung (52) zum Aufnehmen eines Stabs (100),
ein Halteelement (6), das eingerichtet ist, zumindest teilweise in dem Aufnahmeraum (54) positioniert zu werden und das radial expandierbar und/oder kompressibel ist, um das Halten des Kopfes (3) zu ermöglichen, der durch die Öffnung (55) eingesetzt ist;
wobei das Halteelement (6) benachbart zu der Öffnung (55) durch einen ersten Eingriffsabschnitt (61) in Position gehalten wird, welcher mit einer Eingriffsstruktur (56) in Eingriff tritt, die an oder in dem Aufnahmeraum (44) des Aufnahmeteils (5) vorgesehen ist;
ein Verriegelungselement (7), das eingerichtet ist, zumindest teilweise in dem Aufnahmeraum (54) angeordnet zu werden, wobei das Verriegelungselement (7) von einer ersten Position, in welcher es dem benachbart zu der Öffnung (55) durch die Eingriffsstruktur (56) in Position gehaltenen Halteelement (6) ermöglicht wird, zu expandieren und den eingesetzten Kopf (3) freizugeben, hin zu einer zweiten Position bewegbar ist, in welcher eine radiale Expansion des durch die Eingriffsstruktur (56) benachbart zu der Öffnung (55) in Position gehaltenen Halteelements (6) verhindert wird, um einer Freigabe des eingesetzten Kopfs (3) vorzubeugen;
wobei das Halteelement (6) ferner einen zweiten Eingriffsabschnitt (62) zum Eingriff durch das Verriegelungselement (7) aufweist, wenn es in die zweite Position bewegt wird, sodass eine Expansion des Halteelements (6) verhindert wird;
wobei der in dem Aufnahmeraum (54) aufgenommene Kopf (3) verriegelt werden kann, wenn sich das Verriegelungselement (7) in der zweiten Position befindet, wobei das Verriegelungselement (7) eine Druckkraft direkt auf den Kopf (3) ausübt, um den Kopf (3) gegen das Halteelement (6) zu drücken.

2. Eine polyaxiale Knochenverankerungsvorrichtung, umfassend:
ein Knochenverankerungselement (1') mit einem Schaft (2) zum Verankern in einem Knochen und einem Kopf (3'), und eine Kopplungsanordnung zum Koppeln eines Stabs mit dem Knochenverankerungselement, wobei die Kopplungsanordnung (4') umfasst:
ein Aufnahmeteil (5) mit einem ersten Ende (5a), einem zweiten Ende (5b), einer sich durch das erste Ende (5a) und das zweite Ende (5b) erstreckenden Mittenachse (C), einem Aufnahmeraum (54) zum Aufnehmen des Kopfes (3') eines Verankerungselements (1'), wobei der Aufnahmeraum eine Öffnung (55) an dem zweiten Ende (5b) besitzt, der so bemessen ist, dass er das Einsetzen des Kopfes (3') erlaubt, und einer sich von dem Aufnahmeraum zu dem ersten Ende (5a) erstreckenden Bohrung (51), und einer Ausnehmung (52) zum Aufnehmen eines Stabs (100),
ein Halteelement (6), das eingerichtet ist, zumindest teilweise in dem Aufnahmeraum (54) positioniert zu werden und das radial expandierbar und/oder kompressibel ist, um das Halten des Kopfes (3') zu ermöglichen, der durch die Öffnung (55) eingesetzt ist;
wobei das Halteelement (6) benachbart zu der Öffnung (55) durch einen ersten Eingriffsabschnitt (61) in Position gehalten wird, welcher mit einer Eingriffsstruktur (56) in Eingriff tritt, die an oder in dem Aufnahmeraum (44) des Aufnahmeteils (5) vorgesehen ist;
ein Verriegelungselement (7'), das eingerichtet ist, zumindest teilweise in dem Aufnahmeraum (54) angeordnet zu werden, wobei das Verriegelungselement (7, 7') von einer ersten Position, in welcher es dem benachbart zu der Öffnung (55) durch die Eingriffsstruktur (56) in Position gehaltenen Halteelement (6) ermöglicht wird, zu expandieren und den eingesetzten Kopf (3') freizugeben, hin zu einer zweiten Position bewegbar ist, in welcher eine radiale Expansion des durch die Eingriffsstruktur (56) benachbart zu der Öffnung (55) in Position gehaltenen Halteelements (6) verhindert wird, um einer Freigabe des eingesetzten Kopfs (3) vorzubeugen;
wobei das Halteelement (6) ferner einen zweiten Eingriffsabschnitt (62) zum Eingriff durch das Verriegelungselement (7') aufweist, wenn es in die zweite Position bewegt wird, sodass eine Expansion des Halteelements (6) verhindert wird;
wobei der in dem Aufnahmeraum (54) aufgenommene Kopf (3') verriegelt werden kann, wenn sich das Verriegelungselement (7') in der zweiten Position befindet, wobei der Stab (100) eine Druckkraft direkt auf den Kopf (3') ausübt, um den Kopf (3') gegen das Halteelement (6) zu drücken.

3. Die polyaxiale Knochenverankerungsvorrichtung gemäß Anspruch 1 oder 2, wobei das Halteelement (6) eine offene oder geschlossene Ringform festlegt.

4. Die polyaxiale Knochenverankerungsvorrichtung gemäß einem der Ansprüche 1-3, wobei das Halteelement (6) einen oder mehrere Schlitze (63) aufweist, um es dem Halteelement (6) zu erlauben, expandiert und/oder komprimiert zu werden.

5. Die polyaxiale Knochenverankerungsvorrichtung gemäß einem der Ansprüche 1-4, wobei der erste Eingriffsabschnitt (62) des Halteelements (6) eine Ringform oder eine Ringsegmentform besitzt.

6. Die polyaxiale Knochenverankerungsvorrichtung gemäß einem der Ansprüche 1-5, wobei die Eingriffsstruktur (56), die an oder in dem Aufnahmeraum (54) vorgesehen ist, eine an einer Innenwand des Aufnahmeraums (54) ausgebildete Ausnehmung ist.

7. Die polyaxiale Knochenverankerungsvorrichtung gemäß Anspruch 6, wobei die Ausnehmung eine Form einer ringförmigen Nut besitzt.

8. Die polyaxiale Knochenverankerungsvorrichtung gemäß einem der Ansprüche 1-7, wobei der zweite Eingriffsabschnitt des Halteelements (6) eine zylindrische oder konische Form besitzt, die koaxial zu der Mittenachse (C) ist, wenn das Halteelement (6) in dem Aufnahmeraum (54) in Position gehalten ist.

9. Die polyaxiale Knochenverankerungsvorrichtung gemäß einem der Ansprüche 1-8, wobei das Halteelement (6) ferner aufweist: einen hohlen, sphärischen, segmentförmigen Abschnitt (64), der eingerichtet ist, den Kopf (3, 3') darin aufzunehmen.

10. Die polyaxiale Knochenverankerungsvorrichtung gemäß einem der Ansprüche 1-8, wobei das Verriegelungselement (7, 7') umfasst:
eine äußere, zumindest teilweise zylindrische Oberfläche (71), die so bemessen ist, dass sie in gleitenden Eingriff mit einer Innenwand des Aufnahmeraums (54) ist, wenn sie darin zumindest zwischen den ersten und zweiten Positionen aufgenommen ist; und
einen eine Ausnehmung (73) aufweisenden inneren Hohlraum (72) mit einer Innenwand (74), die eingerichtet ist, mit dem zweiten Eingriffsabschnitt (62) des Halteelements (6) in Eingriff zu treten, welches das Halteelement (6) vor einer Expansion bewahrt, wenn sich das Verriegelungselement (7, 7') in der zweiten Position befindet.

11. Die polyaxiale Knochenverankerungsvorrichtung gemäß Anspruch 10, wobei das Verriegelungselement (7) ein Druckelement ist, welches angepasst ist, eine Druckkraft von einem Befestigungselement (9) das in der Bohrung (51) des Aufnahmeteils (5) an dem ersten Ende (5a) angebracht wird, und/oder von einem Stab (100), welcher in der Ausnehmung (52) des Aufnahmeteils (5) aufgenommen ist, zu empfangen und in Richtung auf den Kopf (3) zu übertragen, um den Kopf (3) fest zu verriegeln.

12. Die polyaxiale Knochenverankerungsvorrichtung gemäß Anspruch 11, wobei der innere Hohlraum (72) des Verriegelungselements (7) ferner eine innere, hohle, sphärische, Segmentförmige Ausnehmung (74) umfasst, die eingerichtet ist, den Kopf (3, 3') aufzunehmen.

13. Die polyaxiale Knochenverankerungsvorrichtung gemäß einem der Ansprüche 1-12, wobei das Verriegelungselement (7, 7') einen Stabaufnahmeabschnitt (75) umfasst, der eingerichtet ist, den Stab (100) aufzunehmen.

14. Die polyaxiale Knochenverankerungsvorrichtung gemäß einem der Ansprüche 1-12, wobei das Verriegelungselement (7,7') eine erste Eingriffsschulter (77) umfasst, die eingerichtet ist, mit einer zweiten Eingriffsschulter (57) in Eingriff zu treten, die in der Bohrung (51) oder dem Aufnahmeraum (54) vorgesehen ist, wenn sich das Verriegelungselement (7,7') in der zweiten Position befindet.

15. Die polyaxiale Knochenverankerungsvorrichtung gemäß einem der Ansprüche 1-14, wobei das Halteelement (6) und/oder das Verriegelungselement (7, 7') eingerichtet sind, sodass sie in die Bohrung (51) und den Aufnahmeraum (54) des Aufnahmeteils (5,5') von dem ersten Ende (5a) her einsetzbar sind.

16. Die polyaxiale Knochenverankerungsvorrichtung gemäß Anspruch 2, wobei der Kopf (3') des Knochenverankerungselement eine sphärischen Endoberfläche mit einer Eingriffsausnehmung (31') für einen Schrauber umfasst, wobei die Ausnehmung eine Vielzahl von Flügelabschnitten (34) umfasst, die sich in einer spiralähnlichen Weise von einem Mittelpunkt der freien Endoberfläche her erstrecken; und
das Halteelement (6') und das Verriegelungselement (7') so ausgeformt sind, dass der Kopf (3') sich teilweise da hindurch erstrecken kann, um direkt durch den eingesetzten Stab (100) eingegriffen zu werden.

## Revendications

1. Dispositif d'ancrage osseux polyaxial comprenant :
un élément d'ancrage osseux (1) ayant une tige (2) destinée à être ancrée à un os et une tête (3), et
un assemblage d'accouplement pour accoupler une barre à l'élément d'ancrage osseux, l'ensemble d'accouplement (4) comprenant :
une partie de réception (5) ayant une première extrémité (5a), une deuxième extrémité (5b), un axe central (C) s'étendant à travers la première extrémité (5a) et la deuxième extrémité (5b), un espace de logement (54) pour recevoir la tête (3) d'un élément d'ancrage (1), l'espace de logement ayant une ouverture (55) au niveau de la deuxième extrémité (5b) dimensionnée de manière à permettre l'insertion de la tête (3) et un alésage (51) s'étendant depuis l'espace de logement jusqu'à la première extrémité (5a), et un renfoncement (52) pour recevoir une barre (100),
un élément de retenue (6) configuré pour être positionné au moins en partie dans l'espace de logement (54) et pouvant être écarté et/ou comprimé radialement de manière à permettre la rétention de la tête (3) qui est insérée à travers l'ouverture (55) ;
l'élément de retenue (6) étant retenu en position à côté de l'ouverture (55) par une première portion d'engagement (61) qui s'engage avec une structure d'engagement (56) prévue au niveau de ou dans l'espace de logement (54) de la partie de réception (5) ;
un élément de verrouillage (7) qui est configuré pour être disposé au moins en partie dans l'espace de logement (54), l'élément de verrouillage (7) pouvant être déplacé d'une première position dans laquelle l'élément de retenue (6), retenu en position à côté de l'ouverture (55) par la structure d'engagement (56), peut être écarté et peut libérer la tête insérée (3), dans une deuxième position dans laquelle l'écartement radial de l'élément de retenue (6), retenu en position côté de l'ouverture (55) par la structure d'engagement (56), est empêché de manière à empêcher la libération de la tête insérée (3) ;
l'élément de retenue (6) comportant en outre une deuxième portion d'engagement (62) destinée à être engagée par l'élément de verrouillage (7) lorsqu'il est déplacé dans la deuxième position, de manière à empêcher l'écartement de l'élément de retenue (6) ;
la tête (3) reçue dans l'espace de logement (54) pouvant être verrouillée lorsque l'élément de verrouillage (7) est dans la deuxième position, l'élément de verrouillage (7) exerçant une force de pression directement sur la tête (3) pour presser la tête (3) contre l'élément de retenue (6).

2. Dispositif d'ancrage osseux polyaxial, comprenant :
un élément d'ancrage osseux (1') ayant une tige (2) destinée à être ancrée à un os et une tête (3'), une barre (100), et un ensemble d'accouplements pour accoupler une barre à l'élément d'ancrage osseux, l'ensemble d'accouplement (4') comprenant :
une partie de réception (5') ayant une première extrémité (5a), une deuxième extrémité (5b), un axe central (C) s'étendant à travers la première extrémité (5a) et la deuxième extrémité (5b), un espace de logement (54) pour recevoir une tête (3') d'un élément d'ancrage (1'), l'espace de logement ayant une ouverture (55) au niveau de la deuxième extrémité (5b) dimensionnée de manière à permettre l'insertion de la tête (3') et un alésage (51) s'étendant depuis l'espace de logement jusqu'à la première extrémité (5a), et un renfoncement (52) pour recevoir une barre (100),
un élément de retenue (6) configuré pour être positionné au moins en partie dans l'espace de logement (54) et pouvant être écarté et/ou comprimé radialement de manière à permettre la rétention de la tête (3') qui est insérée à travers l'ouverture (55) ;
l'élément de retenue (6) étant retenu en position à côté de l'ouverture (55) par une première portion d'engagement (61) qui s'engage avec une structure d'engagement (56) prévue au niveau de ou dans l'espace de logement (54) de la partie de réception (5') ;
un élément de verrouillage (7') qui est configuré pour être disposé au moins en partie dans l'espace de logement (54), l'élément de verrouillage (7, 7') pouvant être déplacé d'une première position dans laquelle l'élément de retenue (6), retenu en position à côté de l'ouverture (55) par la structure d'engagement (56), peut être écarté et peut libérer la tête insérée (3'), dans une deuxième position dans laquelle l'écartement radial de l'élément de retenue (6), retenu en position côté de l'ouverture (55) par la structure d'engagement (56), est empêché de manière à empêcher la libération de la tête insérée (3') ;
l'élément de retenue (6) comprenant en outre une deuxième portion d'engagement (62) destinée à être engagée par l'élément de verrouillage (7') lorsqu'il est déplacé dans la deuxième position de manière à empêcher l'écartement de l'élément de retenue (6) ;
la tête (3') reçue dans l'espace de logement (54) pouvant être verrouillée lorsque l'élément de verrouillage (7') est dans la deuxième position, la barre (100) exerçant une force de pression directement sur la tête (3') pour presser la tête (3') contre l'élément de retenue (6).

3. Dispositif d'ancrage osseux polyaxial selon la revendication 1 ou 2, dans lequel l'élément de retenue (6) définit une forme annulaire ouverte ou fermée.

4. Dispositif d'ancrage osseux polyaxial selon l'une quelconque des revendications 1 à 3, dans lequel l'élément de retenue (6) comprend une ou plusieurs fentes (63) afin de permettre à l'élément de retenue (6) d'être écarté et/ou comprimé.

5. Dispositif d'ancrage osseux polyaxial selon l'une quelconque des revendications 1 à 4, dans lequel la première portion d'engagement (62) de l'élément de retenue (6) présente une forme annulaire ou une forme de segment annulaire.

6. Dispositif d'ancrage osseux polyaxial selon l'une quelconque des revendications 1 à 5, dans lequel la structure d'engagement (56) prévue au niveau de ou dans l'espace de logement (54) est un renfoncement formé au niveau d'une paroi interne de l'espace de logement (54).

7. Dispositif d'ancrage osseux polyaxial selon la revendication 6, dans lequel le renfoncement présente la forme d'une gorge annulaire.

8. Dispositif d'ancrage osseux polyaxial selon l'une quelconque des revendications 1 à 7, dans lequel la deuxième portion d'engagement de l'élément de retenue (6) présente une forme cylindrique ou conique qui est coaxiale avec l'axe central (C) lorsque l'élément de retenue (6) est retenu en position dans l'espace de logement (54).

9. Dispositif d'ancrage osseux polyaxial selon l'une quelconque des revendications 1 à 8, dans lequel l'élément de retenue (6) comporte en outre : une portion creuse en forme de segment sphérique (64) configurée pour recevoir la tête (3, 3').

10. Dispositif d'ancrage osseux polyaxial selon l'une quelconque des revendications 1 à 8, dans lequel l'élément de verrouillage (7, 7') comprend :
une surface extérieure au moins en partie cylindrique (71) dimensionnée de manière à être en engagement coulissant avec une paroi interne de l'espace de logement (54) tout en étant reçue dans celui-ci au moins entre la première et la deuxième position ; et
une cavité interne (72) comportant un renfoncement (73) ayant une paroi interne (74) configurée pour s'engager avec la deuxième portion d'engagement (62) de l'élément de retenue (6), ce qui empêche l'écartement de l'élément de retenue (6) lorsque l'élément de verrouillage (7, 7') est dans la deuxième position.

11. Dispositif d'ancrage osseux polyaxial selon la revendication 10, dans lequel l'élément de verrouillage (7) est un élément de pression qui est prévu pour recevoir et transférer une force de pression depuis un élément de fixation (9), qui est attaché à l'alésage (51) de la partie de réception (5) au niveau de la première extrémité (5a), et/ou une barre (100), qui est reçue dans le renfoncement (52) de la partie de réception (5), à la tête (3) afin de verrouiller par serrage la tête (3).

12. Dispositif d'ancrage osseux polyaxial selon la revendication 11, dans lequel la cavité interne (72) de l'élément de verrouillage (7) comprend en outre un renfoncement interne creux en forme de segment sphérique (74) configuré pour recevoir la tête (3, 3') .

13. Dispositif d'ancrage osseux polyaxial selon l'une quelconque des revendications 1 à 12, dans lequel l'élément de verrouillage (7, 7') comprend une portion de réception de barre (75) configurée pour recevoir la barre (100).

14. Dispositif d'ancrage osseux polyaxial selon l'une quelconque des revendications 1 à 12, dans lequel l'élément de verrouillage (7, 7') comprend un premier épaulement d'engagement (77) configuré pour s'engager avec un deuxième épaulement d'engagement (57) prévu dans l'alésage (51) ou l'espace de logement (54), lorsque l'élément de verrouillage (7, 7') est dans la deuxième position.

15. Dispositif d'ancrage osseux polyaxial selon l'une quelconque des revendications 1 à 14, dans lequel l'élément de retenue (6) et/ou l'élément de verrouillage (7, 7') sont configurés de manière à pouvoir être insérés dans l'alésage (51) et l'espace de logement (54) de la partie de réception (5, 5') depuis la première extrémité (5a).

16. Dispositif d'ancrage osseux polyaxial selon la revendication 2, dans lequel
la tête (3') de l'élément d'ancrage osseux comprend une surface d'extrémité sphérique avec un renfoncement d'engagement (31') pour un dispositif d'enfoncement, le renfoncement comprenant une pluralité de portions d'aile (34) qui s'étendent en spirale à partir d'un centre de la surface d'extrémité libre ; et
l'élément de retenue (6') et l'élément de verrouillage (7') sont formés de manière à ce que la tête (3') puisse s'étendre en partie à travers eux afin d'être directement engagée par la barre insérée (100).
